# EUROPÄISCHE PATENTSCHRIFT

(11) **EP 2 208 779 B1**
(45) Veröffentlichungstag und Bekanntmachung des Hinweises auf die Patenterteilung: **05.04.2017**
(21) Anmeldenummer: 09000700.6
(22) Anmeldetag: 20.01.2009
(51) Int. Cl.: C12M 1/42, C12M 3/00

(54) **Behältnis mit mehreren Reaktionsräumen und Elektroden**
Container with multiple reaction chambers and electrodes
Récipient doté de plusieurs espaces de réaction et électrodes

(43) Veröffentlichungstag der Anmeldung: 21.07.2010
(73) Patentinhaber: Lonza Cologne GmbH, 50829 Köln (DE)
(72) Erfinder: Altrogge, Ludger, 53894 Mechemich (DE); Gleissner, timo, 53879 Euskirchen (DE); Heinze, Andreas, 50739 Köln (DE); Müller-Hartmann, herbert, 50937 Köln (DE); Wirth, Andreas, 49525 Lengerich (DE)
(74) Vertreter: Remus, Alvaro Johannes

(56) Entgegenhaltungen:
- EP-A- 1 577 378
- WO-A-02/05293
- WO-A-2005/044983
- WO-A-2007/094947
- US-A- 5 183 744
- US-A1- 2007 231 873
- US-B1- 6 878 538

## Beschreibung

Die Erfindung betrifft ein Behältnis mit mindestens drei Reaktionsräumen, die jeweils mindestens ein Elektrodenpaar zum Anlegen einer elektrischen Spannung zur Erzeugung eines elektrischen Feldes innerhalb des Reaktionsraums aufweisen und die geometrisch in mindestens einer Reihe angeordnet und/oder elektrisch in mindestens einer Reihe geschaltet sind, wobei mindestens eine Elektrode eines Reaktionsraums eine gemeinsame Elektrode mit mindestens einem anderen Reaktionsraum ist. Die Erfindung betrifft ferner einen Deckel für dieses Behältnis. Die Erfindung betrifft auch ein Verfahren zur Herstellung des genannten Behältnisses, bei dem pro Reaktionsraum mindestens zwei Bereiche aus einem leitfähigen Polymer in eine Spritzgussform gespritzt werden und um die mindestens zwei Bereiche herum ein die Reaktionsräume begrenzender Wandbereich aus einem nicht-leitfähigen Polymer gespritzt wird.

Die vorliegende Erfindung wird durch die beigefügten Ansprüche definiert. Jeder Gegenstand in der Anmeldung, der als "Erfindung", "Ausführungsbeispiel", "Aspekt", usw. identifiziert wird, der den Schutzumfang der Erfindung überschreitet, wie er durch die Patentansprüche definiert ist, ist nicht Teil der beanspruchten Erfindung, sondern dient nur als Hintergrundinformation zum besseren Verständnis der Erfindung.

Mit Elektroden versehene Behältnisse werden insbesondere bei Anwendungen eingesetzt, bei denen der Reaktionsansatz mit einem elektrischen Spannungsimpuls beaufschlagt werden muss, wie beispielsweise die Elektroporation, Elektrofusion und Elektrostimulation von lebenden Zellen. Solche Behältnisse können auch mehrere Reaktionsräume aufweisen, wobei jeder Reaktionsraum mit Elektroden versehen sein kann. Diese Behältnisse bezeichnet man in der Regel als Mehrlochplatten, Mikrotiterplatten oder Multiwells. Sie werden vor allem bei biochemischen und pharmazeutischen Anwendungen verwendet, bei denen eine Vielzahl von Reaktionsansätzen gleichzeitig getestet werden muss. Dabei ist das Bestreben eine möglichst große Anzahl von Reaktionsräumen, beispielsweise 384, zur Verfügung zu stellen insbesondere bei HT-Analysen (HT = high throughput) zu erkennen, da hier eine Vielzahl von Proben in möglichst kurzer Zeit getestet werden soll.

Die bekannten Behältnisse bestehen üblicherweise aus mehreren Reaktionsräumen, die jeweils zwei Elektroden aufweisen, welche mit dem Reaktionsansatz, beispielsweise einer Zellsuspension, im Reaktionsraum in Kontakt stehen. Die beiden Elektroden eines Reaktionsraums erzeugen bei Anlegen einer elektrischen Spannung im Inneren des Reaktionsraums ein elektrisches Feld und einen Stromfluss, wobei sie beispielsweise bei Gleichstrom unterschiedliche Potentiale und/oder Polaritäten aufweisen. Die Elektroden gleicher Polarität, d. h. beispielsweise alle Kathoden und/oder alle Anoden, verschiedener Reaktionsräume sind dabei entweder einstückig ausgebildet oder elektrisch miteinander gekoppelt, so dass sie über einen gemeinsamen elektrischen Kontakt mit der Spannungsquelle verbunden werden können.

Die Beaufschlagung der Reaktionsräume solcher Behältnisse mit Spannungsimpulsen erfolgt mittels besonderer Schaltungsanordnungen, die eine oder zwei Speichereinrichtung(en) zum Speichern elektrischer Ladungen umfassen. Bei den Speichereinrichtungen handelt es sich jeweils um Kondensatoren, die auf eine vorbestimmte elektrische Ladung aufgeladen werden und durch gezielte Entladung definierte Spannungsimpulse abgeben können. Die Speichereinrichtungen sind mit elektrischen Schaltern verbunden, beispielsweise Leistungshalbleitern, über welche die gezielte Entladung der Speichereinrichtungen geschaltet wird. Die Verwendung von zwei Speichereinrichtungen ermöglicht die Abgabe von zwei kurz aufeinander folgenden oder ineinander übergehenden Spannungsimpulsen, was bei der Elektroporation von bestimmten Zelltypen von Vorteil sein kann. Zur elektrischen Kontaktierung der Elektroden der Behältnisse dienen in der Regel Kontaktstifte, die auf Armen oder Platten angeordnet sind und manuell oder automatisch mit den Elektroden in Kontakt gebracht werden.

Aus der EP-A-1 577 378 ist ein Behältnis mit mehreren Reaktionsräumen bekannt, das mehrere Module umfasst. Jedes Modul weist dabei zwei parallel angeordneten Reihen von Reaktionsräumen auf, die jeweils ein aus einer ersten und einer zweiten Elektrode bestehendes Elektrodenpaar zum Anlegen einer elektrischen Spannung zur Erzeugung eines elektrischen Feldes innerhalb des Reaktionsraums aufweisen. Die auf der gleichen Seite der unterschiedlichen Reaktionsräume einer Reihe angeordneten ersten Elektroden sind elektrisch leitfähig gekoppelt, während die zweiten Elektroden eines Reaktionsraumes elektrisch separat anschließbar sind. Dabei sind auch die gegenüberliegend angeordneten ersten Elektroden unterschiedlicher Reaktionsräume benachbarter Reihen elektrisch leitfähig gekoppelt. Bei diesem bekannten Behältnis sind alle Reaktionsräume einzeln bzw. individuell adressierbar, d. h. jeder einzelne Reaktionsraum kann unabhängig von den anderen Reaktionsräumen mit einem Spannungsimpuls beaufschlagt werden.

Aus der US-A-5 183 744 ist ferner ein Behältnis mit mehreren Reaktionsräumen bekannt, bei dem die einzelnen Reaktionsräume ebenfalls einzeln adressierbar sind. Jeder einzelne Reaktionsraum dieses bekannten Behältnisses ist mit zwei Elektroden versehen, wobei jeweils eine der Elektroden eines Elektrodenpaars mit den entsprechenden Elektroden der anderen Reaktionsräume einer Reihe von Reaktionsräumen elektrisch gekoppelt ist. Die jeweils andere Elektrode des Elektrodenpaars eines Reaktionsraums wird separat elektrisch kontaktiert, so dass jeder einzelne Reaktionsraum individuell mit einem Spannungsimpuls beaufschlagt werden kann. Die US-A-5 183 744 offenbart ferner ein Behältnis mit mehreren Reaktionsräumen, bei dem die Elektroden der einzelnen Reaktionsräume matrixartig miteinander verbunden sind. Die Matrix wird dabei von sich kreuzenden Leiterbahnen gebildet, wobei die Leiterbahnen an den jeweiligen Kreuzungspunkten durch eine isolierende Schicht voneinander getrennt sind. Die einzelnen Elektroden sind über Kontaktpunkte mit den Leiterbahnen verbunden, so dass theoretisch jeder einzelne Reaktionsraum adressierbar ist. Wird nämlich an zwei sich kreuzende Leiterbahnen eine Spannung angelegt, so wird theoretisch nur der Reaktionsraum mit der Spannung beaufschlagt, der mit diesen beiden Leiterbahnen verbunden ist. Es hat sich in der Praxis jedoch herausgestellt, dass bei einer solchen Matrix parasitäre Ströme entstehen, so dass auch durch andere Reaktionsräume ungewollt elektrischer Strom fließt. Eine solche Lösung hat also den Nachteil, dass unerwünschte Nebeneffekte auftreten, welche die Effizienz und die Reproduzierbarkeit der mit solchen Behältnissen durchgeführten Verfahren erheblich beeinträchtigen.

Die WO 2005/044983 A2 offenbart ebenfalls ein Behältnis mit mehreren Reaktionsräumen, bei dem jeder Reaktionsraum ein Elektrodenpaar zum Anlegen einer elektrischen Spannung aufweist. Die Elektroden sind dabei, wie bereits aus der US-A-5 183 744 bekannt, durch sich kreuzende Leiterbahnen matrixartig miteinander verbunden. Auch hier lassen sich folglich parasitäre Ströme und somit unerwünschte Nebeneffekte nicht vermeiden.

Die US 2007/0231873 A1 offenbart ein Behältnis mit mehreren Reaktionsräumen, bei dem jeder einzelne Reaktionsraum mit zwei gegenüberliegend angeordneten Elektroden versehen ist. Allerdings sind bei diesem Behältnis die einzelnen Reaktionsräume nicht individuell adressierbar, sondern es können lediglich einzelne Gruppen, die jeweils aus mehreren Reaktionsräumen bestehen, gleichzeitig mit einem Spannungsimpuls beaufschlagt werden.

Aus der WO 2007/094947 A2 ist ferner ein Behältnis mit mehreren Reaktionsräumen bekannt, bei dem jeder Reaktionsraum zwei gegenüberliegend angeordnete Elektroden aufweist. Allerdings hat auch dieses bekannte Behältnis den Nachteil, dass hier die einzelnen Reaktionsräume nicht individuell, sondern lediglich in Gruppen adressierbar sind.

Alle bekannten Behältnisse haben den Nachteil, dass entweder die Reaktionsräume nicht separat adressierbar sind oder die Behältnisse insgesamt eine hohe Anzahl an Elektroden aufweisen, wobei die Anzahl der Elektroden doppelt so hoch ist wie die Anzahl der Reaktionsräume, Hieraus ergibt sich bei gegebenen Dimensionen des Behältnisses ein verringertes aktives und passives Volumen der einzelnen Reaktionsräume.

Es ist Aufgabe der Erfindung, ein Behältnis mit individuell adressierbaren Reaktionsräumen der eingangs genannten Art zur Verfügung zu stellen, das bei gegebenen Dimensionen des Behältnisses ein erhöhtes aktives und passives Volumen der einzelnen Reaktionsräume aufweist.

Erfindungsgemäß wird die Aufgabe durch ein Behältnis der eingangs genannten Art gelöst, bei dem n + x Elektroden vorgesehen sind, wobei n die Anzahl der Reaktionsräume mit n ≥ 3 und x die Anzahl der Reihen mit x ≥ 1 ist. Durch die Reduzierung der Elektrodenanzahl wird das aktive Volumen der einzelnen Reaktionsräume bei gegebenen Dimensionen des Behältnisses maximiert. Als aktives Volumen wird damit im Sinne der Erfindung das Volumen eines Reaktionsraums bezeichnet, das sich zwischen den Elektroden befindet, d. h. innerhalb dessen im Wesentlichen die eigentlichen Reaktionen bzw. Prozesse ablaufen. "Reihe" im Sinne der Erfindung bedeutet, dass die Reaktionsräume geometrisch in mindestens einer Reihe, Zeile oder Spalte angeordnet und/oder die Reaktionsräume bzw. mindestens eine ihrer Elektroden elektrisch in mindestens einer Reihe geschaltet sind. Durch den erfindungsgemäßen Aufbau des Behältnisses kann beispielsweise die Zahl der erforderlichen Elektroden für ein Behältnis mit 384 Reaktionsräumen von beispielsweise 768 auf 408 reduziert werden. Durch die reduzierte Elektrodenanzahl kann ferner Material eingespart und der apparative Aufwand auf Seiten des Spannungsimpulsgebers und der Kontaktierungseinrichtung erheblich reduziert werden. Darüber hinaus verringert sich bei der Herstellung des erfindungsgemäßen Behältnisses im Spritzgussverfahren der Aufwand bei der Herstellung des Werkzeugs.

In vorteilhafter Ausgestaltung der Erfindung ist vorgesehen, dass innerhalb der Reihe mindestens eine gemeinsame Elektrode zumindest teilweise zwischen zwei benachbarten Reaktionsräumen angeordnet ist. Durch diese alternierende geometrische Anordnung der Elektroden wird der Abstand zwischen den Elektroden bzw. diesen zugeordneten Kontaktelementen maximiert, so dass die elektrische Sicherheit deutlich erhöht werden kann. Dabei können die Elektroden auch versetzt und/oder in Bezug auf die Längsachse des Behältnisses zickzack-förmig angeordnet sein.

In vorteilhafter Ausgestaltung der Erfindung ist vorgesehen, dass die Elektroden mit Kontaktelementen versehen sind, die zum Anlegen der elektrischen Spannung elektrisch kontaktierbar sind. Vorzugsweise ist insbesondere die gemeinsame Elektrode der beiden Reaktionsräume mit einem Kontaktelement versehen. Mittels der Kontaktelemente sind die Elektroden leicht zu kontaktieren, wobei für die Kontaktelemente ein Material gewählt werden kann, das einen optimalen Stromfluss in die Elektroden gewährleistet. Jedes Kontaktelement des Behältnisses weist vorzugsweise den größtmöglichen Abstand zu den jeweils benachbarten Kontaktelementen auf.

In besonders vorteilhafter Ausgestaltung der Erfindung ist vorgesehen, dass die Kontaktelemente, mit denen die Elektroden eines Reaktionsraums versehen sind, auf gegenüberliegenden Seiten dieses Reaktionsraums angeordnet sind. Vorzugsweise sind die Kontaktelemente dabei diagonal gegenüberliegend und/oder versetzt bzw. in Bezug auf die Länge des Behältnisses zickzackförmig angeordnet, so dass sie den größtmöglichen Abstand zueinander einnehmen. Auf diese Weise wird verhindert, dass zwischen zwei benachbarten Kontaktelementen elektrische Überschläge auftreten können. Darüber hinaus wird hierdurch das passive Volumen der einzelnen Reaktionsräume bei gegebenen Gesamtvolumen maximiert. Als passives Volumen wird damit im Sinne der Erfindung das Volumen eines Reaktionsraums bezeichnet, das sich nicht zwischen den Elektroden befindet, d. h. innerhalb dessen eigentlich im Wesentlichen keine Reaktionen bzw. elektrisch stimulierte Prozesse ablaufen. Vorzugsweise weist die Kontaktfläche der Kontaktelemente zu den Elektroden, die sich an oder innerhalb der Elektroden befindet, eine Fläche von mindestens 5 mm², vorzugsweise mindestens 6 mm², besonders bevorzugt mindestens 7 mm², insbesondere mindestens 8 mm², auf.

Die Kontaktelemente können in vorteilhafter Ausgestaltung der Erfindung von den Elektroden teilweise umschlossen sein, wobei mindestens 10 %, insbesondere mindestens 20 % oder mindestens 30 %, vorzugsweise mindestens 40 %, besonders bevorzugt mindestens 50 %, der Länge der Kontaktelemente innerhalb der Elektroden liegen kann.

Vorzugsweise sind die Kontaktelemente stift-, nadel- oder schraubenförmig ausgebildet und/oder weisen einen zumindest annähernd runden Querschnitt auf. Diese Form der Kontaktelemente erleichtert bei der Herstellung der erfindungsgemäßen Behältnisse die Befestigung der Kontaktelemente in den Elektroden und gewährleistet einen hervorragenden elektrischen Kontakt. Die Kontaktelemente können beispielsweise vom leitfähigen Material der Elektroden im Spritzgussverfahren umspitzt oder in das leitfähige Elektrodenmaterial eingeschraubt werden. Zur weiteren Verbesserung des elektrischen Kontakts können die Kontaktelemente auch gerändelt oder anderweitig aufgeraut sein. Auch zumindest teilweise abgeflachte Querschnitte können in besonderen Fällen vorteilhaft sein, z. B. um die Verankerung in den Elektroden zu verbessern.

In vorteilhafter Ausgestaltung der Erfindung weisen die Reaktionsräume im oberen Bereich eine Öffnung auf, wobei die Elektroden im oberen Bereich kontaktierbar sind. Durch die Kontaktierung von oben bleibt die Unterseite des Behältnisses zugänglich, so dass beispielsweise optische Messungen durchgeführt werden können. Darüber hinaus kann bei dieser Ausführungsform das Behältnis über die Unterseite, insbesondere über die Elektroden, leicht gekühlt werden, was insbesondere bei Anwendungen, bei denen mit Spannungsimpulsen in schneller Folge bzw. mit hohem Durchsatz und mit sehr hoher Amplitude gearbeitet wird, aufgrund der starken Wärmeentwicklung von Vorteil ist.

In weiterer vorteilhafter Ausgestaltung der Erfindung ist vorgesehen, dass die Kontaktelemente an der Oberseite der Elektroden angeordnet sind und/oder nach oben aus den Elektroden herausragen, so dass die Elektroden leicht von oben kontaktiert werden können. Dies hat den Vorteil, dass der Boden frei bleibt und somit die Durchsicht zur Analyse des Reaktionsrauminhalts möglich ist. Falls für spezielle Anwendungen ein anderer als der Standardboden erforderlich ist, kann der Boden, beispielsweise mechanisch oder chemisch, entfernt und durch einen Boden aus einem anderen Material ersetzt werden (z. B. Folie, Glas), ohne die Funktionalität des erfindungsgemäßen Behältnisses zu verlieren.

In besonders vorteilhafter Ausgestaltung der Erfindung ist vorgesehen, dass mindestens eine, vorzugsweise jede, Reihe ein Modul bildet und dass die Module direkt oder indirekt miteinander verbindbar sind. Durch diesen modularen Aufbau ist es möglich, verschiedene Formate des erfindungsgemäßen Behältnisses sehr flexibel zur Verfügung zu stellen. So können beispielsweise durch die Bereitstellung von Modulen mit 16 Reaktionsräumen Behältnisse mit 96 Reaktionsräumen, bestehend aus 6 Modulen, oder Behältnisse mit 384 Reaktionsräumen, bestehend aus 24 Modulen, durch einfache Kombination der einzelnen Module zur Verfügung gestellt werden. Vorzugsweise werden die einzelnen Module dabei in einem geeigneten Rahmen befestigt, so dass sie lösbar oder unlösbar miteinander verbunden sind und eine Einheit bilden. Der Zusammenbau der Module im Rahmen kann beispielsweise mittels irreversibler Rastmechanismen oder mittels Verklebung erfolgen. Reversibler Zusammenbau der Module im Rahmen mit entsprechenden Rastmechanismen ist ebenfalls möglich. Die Module können auch mittels Ultraschall-Schweißen, Verschraubung, Vernietung, Heißverstemmung oder Verklemmung in dem Rahmen befestigt werden. Die Module bestehen vorzugsweise aus zwei Kunststoffkomponenten, d. h. beispielsweise elektrisch leitfähigem Polycarbonat und transparentem Polycarbonat, sowie Einlege-Stiften, die beispielsweise aus vorzugsweise vernickeltem Messing bestehen können. Das leitfähige Polycarbonat dient zur Herstellung der Elektroden, das transparente Polycarbonat als Basis für den Grundkörper bzw. den Wandbereich. Der Rahmen kann ein Kunststoff-Spritzgussteil sein und beispielsweise aus Polystyrol oder anderen thermoplastischen Kunststoffen bestehen.

Wenn das Behältnis und/oder jeder Reaktionsraum einen Boden aufweist, der zumindest teilweise transparent ist, können beispielsweise optische Überwachungen und/oder Messungen im Innenraum der Reaktionsräume vorgenommen werden. Dabei kann das Behältnis und/oder jeder Reaktionsraum einen Boden aufweisen, der zumindest teilweise aus Glas und/oder Kunststoff besteht.

Um beispielsweise das Anhaften von Zellen zu ermöglichen oder bestimmte optische Messungen durchzuführen, kann der Boden zumindest teilweise beschichtet sein. Die Beschichtung kann dabei künstliche Polymere und/oder Biopolymere umfassen.

In einer besonderen Ausführungsform der Erfindung kann sich der Boden durchgehend unter dem gesamten Behältnis erstrecken.

Vorzugsweise umfasst das erfindungsgemäße Behältnis eine Vielzahl von Reaktionsräumen, vorzugsweise 6, 8, 12, 16, 24, 32, 48, 64, 96, 128, 192, 384, 1536, 3456 oder 6144 Reaktionsräume.

Zumindest eine Elektrode des erfindungsgemäßen Behältnisses kann aus einem Polymer bestehen, das mit einem elektrisch leitfähigen Stoff dotiert ist. Als Dotierung können dabei alle elektrisch leitfähigen Stoffe verwendet werden, beispielsweise Metalle oder leitfähige Kunststoffe. Besonders bevorzugt sind jedoch kohlenstoffhaltige Substanzen, insbesondere Kohlefasern, Graphit, Ruß und/oder Kohlenstoff-Nanotubes. Die Dotierung ist dabei vorzugsweise in einer Konzentration von 20 bis 80 Ges.-% in dem Polymer enthalten.

Die Kontaktelemente bestehen vorzugsweise aus einem Kontaktmaterial, welches bei 23°C einen geringeren spezifischen Widerstand oder Grenzflächenwiderstand als dasjenige Material aufweist, aus dem die Elektroden bestehen. Das Kontaktmaterial kann beispielsweise ein Metall sein und/oder einen spezifischen Widerstand bei 23°C unterhalb von 1 x 10⁻⁵ Ohm·cm, vorzugsweise von 1 x 10⁻⁶ bis 2 x 10⁻⁶ Ohm·cm, aufweisen.

Wenn die Elektroden des erfindungsgemäßen Behältnisses von unten gekühlt werden sollen oder müssen, ist es besonders von Vorteil, wenn die Elektroden nach unten über den Boden des Behältnisses und/oder den Wandbereich der Reaktionsräume hinausragen. Auf diese Weise kann der Kontakt zu einem geeigneten Kühlelement intensiviert werden, so dass der Wärmeübergang optimiert werden kann.

In einer Ausführungsform der Erfindung ist ein Deckel für das erfindungsgemäße Behältnis vorgesehen, welcher eine der Anzahl der Kontaktstellen entsprechende Anzahl von Löchern aufweist. Durch diese erfindungsgemäße Ausgestaltung wird es ermöglicht, die Elektroden des Behältnisses zu kontaktieren, ohne den Deckel abnehmen zu müssen. Die Schutzfunktion des Deckels bleibt also auch während der Durchführung des jeweiligen Verfahrens erhalten.

In besonders vorteilhafter Ausgestaltung der Erfindung ist vorgesehen, dass der Deckel n + x Löcher aufweist, wobei n die Anzahl der Reaktionsräume mit n ≥ 3 und x die Anzahl der Reihen mit x ≥ 1 ist.

In weiterer vorteilhafter Ausgestaltung der Erfindung ist vorgesehen, dass der Deckel auf seiner den Reaktionsräumen zugewandten Seite im Bereich der Ränder der Reaktionsräume Erhebungen aufweist und/oder mit Dichtungsmaterial versehen ist, so dass kein Material bzw. keine Flüssigkeit aus den einzelnen Reaktionsräumen austreten kann. Das ist besonders dann von Vorteil, wenn sehr hohe Ströme durch die Reaktionsräume fließen und es hierdurch zu verstärkter Gasbildung kommt, die zum Spritzen führen kann. Durch die besondere Ausgestaltung des Deckels an der Innenseite können somit insbesondere Kreuzkontaminationen, d. h. Kontaminationen von einem Reaktionsraum zu einem anderen Reaktionsraum, vermieden werden.

Der Deckel kann ein Kunststoff-Spritzgussteil sein und beispielsweise aus transparentem Polystyrol bestehen.

Das erfindungsgemäße Behältnis wird in einem Verfahren der eingangs genannten Art hergestellt, bei dem zunächst die Kontaktelemente in die Spritzgussform eingelegt und dann von mindestens einem der beiden Polymere umspritzt werden. Auf diese Weise kann das Behältnis mit hoher Qualität und Präzision in einem Spritzgussprozess mit hoher Geschwindigkeit hergestellt werden. Das Polymer, mit dem die Kontaktelemente umspritzt werden, ist dabei vorzugsweise das elektrisch leitfähige Polymer. Die beiden Polymere, aus denen das Behältnis im Wesentlichen besteht, können auch im Mehrkomponentenspritzguss gespritzt werden. In diesem Fall werden die beiden Polymere aber nicht als Mischung gespritzt, sondern lediglich in zwei aufeinander folgenden Schritten eines Arbeitsgangs. Durch das Einlegen der Kontaktelemente in das Spritzgusswerkzeug wird dabei die Notwendigkeit eines zusätzlichen Arbeitsgangs vermieden und somit das Herstellungsverfahren vereinfacht und beschleunigt.

Das Spritzgussverfahren für die Herstellung von Mikrotiterplatten ist eine vorteilhafte Methode, um diese in hohen Stückzahlen kostengünstig zu produzieren. Die Verwendung von Einlegeteilen in Spritzgussteilen dient dazu, mechanische oder elektrische Eigenschaften von Spritzgussteilen zu verbessern bzw. zu ermöglichen. Es bietet eine kostengünstige Möglichkeit, da die Kontaktelemente in einem Arbeitsschritt automatisch in das SpritzgussWerkzeug eingelegt werden können. Dies vereinfacht die Herstellung von Multiwellplatten für die Elektroporation und dieser Prozess ist zudem mit hoher Präzision verbunden.

Alternativ zum Umspritzen der Kontaktelemente können diese auch in das leitfähige Elektrodenmaterial eingeschraubt oder eingepresst werden. Es ist ferner möglich, die Kontaktelemente mit einem leitfähigen Kleber in das leitfähige Elektrodenmaterial einzukleben.

In einer weiteren vorteilhaften Ausführungsform können die Elektroden so ausgeformt werden, dass sie an der Oberseite des Behältnisses durch Heißprägen mit Kontaktelementen bzw. Kontaktflächen versehen werden können.

Das erfindungsgemäße Behältnis kann erfindungsgemäß auch in einen Verfahren hergestellt werden, bei dem einzelne Module im Spritzgussverfahren hergestellt werden, die jeweils aus in mindestens einer Reihe angeordneten Reaktionsräumen bestehen und an ihren Schmalseiten Befestigungselemente aufweisen. Dabei kann mindestens ein Modul in einem Rahmenelement befestigt werden, wobei das Rahmenelement für jedes zu befestigende Modul auf zwei gegenüberliegenden Seiten jeweils mindestens ein Befestigungsmittel, vorzugsweise in Form eines Fortsatzes, aufweist, welches mit dem jeweiligen Befestigungselement, vorzugsweise in Form von Ösen, korrespondiert. Vorzugsweise wird das Modul durch Heißverstemmen in dem Rahmenelement befestigt, wobei das Befestigungsmittel in das jeweilige Befestigungselement eingreift, wenn das Modul in das Rahmenelement einlegt wird, und wobei das Befestigungsmittel derart erhitzt wird, dass es zum Formschluss zwischen dem Befestigungsmittel und dem Befestigungselement kommt. Alternativ können die Module aber auch mittels Ultraschallschweißen in dem Rahmenelement befestigt oder mittels eines geeigneten Klebers in das Rahmenelement eingeklebt werden.

In einer besonderen Ausführungsform des erfindungsgemäßen Verfahrens kann der Boden des Wandbereichs vom Behältnis entfernt werden und anschließend ein neuer Boden an dem Behältnis angebracht werden. Auf diese Weise kann das Behältnis flexibel an besondere Erfordernisse angepasst werden. Wenn der neue Boden aus transparentem Material besteht, kann beispielsweise ein Behältnis zur Verfügung gestellt werden, bei dem optische Messungen in den Innenräumen durchgeführt werden können.

Um das erfindungsgemäße Behältnis individuell identifizieren zu können kann es erfindungsgemäß unlösbar mit mindestens einem Transponder verbunden sein, insbesondere einem Transponder zur eindeutigen Identifikation des Behältnisses. Mittels eines geeigneten Lesegeräts können dabei die das jeweilige Behältnis betreffenden Daten ausgelesen und elektronisch verarbeitet werden. Ein zeitaufwendiges manuelles Kennzeichnen oder Beschriften der Behältnisse wird dadurch vermieden, wobei die Information erfindungsgemäß nicht mehr optisch erkennbar sein muss. Dies ist insbesondere von Vorteil, wenn in einer Testreihe eine Vielzahl von Behältnissen verwendet wird, wie es beispielsweise bei Hochdurchsatzverfahren der Fall ist. Mit Hilfe des erfindungsgemäßen Behältnisses können solche Verfahren beschleunigt werden, wobei die Datenerfassung verbessert und vor allem sicherer wird. Darüber hinaus ist die Information schwieriger zu manipulieren, die Prozesssicherheit ist deutlich erhöht und die Rückverfolgbarkeit der Behältnisse ist optimal.

In vorteilhafter Ausgestaltung der Erfindung ist der Transponder in den Wandbereich, der den mindestens einen Reaktionsraum begrenzt bzw. bildet, integriert. Wenn der Wandbereich bzw. Grundkörper des Behältnisses aus spritzfähigem Kunststoff besteht, kann der Transponder beispielsweise während des Produktionsprozesses in den Wandbereich des Behältnisses eingespritzt werden.

Der Transponder ist vorzugsweise RFID-Transponder. Alternativ kann er aber auch beispielsweise ein "One-Wire-Identification"-Tag wie z. B. "iButton" (MAXIM/DALLAS) sein. Der RFID-Transponder umfasst vorzugsweise eine Antenne, einen analogen Schaltkreis, einen digitalen Schaltkreis und/oder einen Speicher.

In Ausgestaltung der Erfindung kann ein solches Behältnis in einem Verfahren hergestellt werden, bei dem das Behältnis im Spritzgussverfahren hergestellt und der Transponder von zumindest einem Polymer umspritzt wird.

Jedes Behältnis kann also erfindungsgemäß mit einem Transponder (Tag) ausgestattet werden, der entweder als Glaskapsel während des Spritzgussprozesses oder anschließend durch Einkleben bzw. Aufbringen in Form eines Aufklebers in oder an dem Behältnis befestigt wird. Das Gerät in dem die Behältnisse prozessiert werden, sollte über eine entsprechende Technik zum Auslesen der Transponder verfügen. Im Gerät folgt dann zur Prozesszeit ein Speichern der Identifikation. Mittels der ausgelesenen Information kann entsprechend des vorher konfigurierten Experiments ein Fehler in der Plattenreihenfolge durch Adaption des Setups zur Laufzeit erfolgen. Die gespeicherten Informationen können zur Auswertung/Korrelation von Fehlerbildern und Zuordnung zu Produktionsbatches verwendet werden. Bei Wiederverwendung bereits prozessierter Behältnisse, kann entsprechend das Experiment gestoppt oder eine Warnung an den Benutzer ausgegeben werden. Das erfindungsgemäße, mit einem Transponder ausgestattete Behältnis kann ferner zur Sequenzprüfung, Rückverfolgung von Reaktionsplatten, Sicherstellung von Prozesssicherheit oder zur Defektvermeidung bei der elektrischen Behandlung zur Analyse von Flüssigkeitsproben verwendet werden.

Die Erfindung wird im Weiteren anhand der Abbildungen beispielhaft näher erläutert.
Figur 1 zeigt eine perspektivische Ansicht einer besonders vorteilhaften Ausführungsform eines erfindungsgemäßen Behältnisses.
Figur 2 zeigt eine Draufsicht auf das erfindungsgemäße Behältnis gemäß Figur 1.
Figur 3 zeigt eine perspektivische Ansicht der Anordnung der Elektroden des Behältnisses gemäß Figur 1.
Figur 4 zeigt einen Querschnitt sowie die Form des Schnittes durch einen Teil des erfindungsgemäßen Behältnisses gemäß der Figur 1.
Figur 5 zeigt eine Draufsicht auf eine besondere Ausführungsform eines aus Modulen zusammengesetzten erfindungsgemäßen Behältnisses mit einem Rahmenelement.
Figur 6 zeigt eine perspektivische Ansicht des Rahmenelements gemäß Figur 5 ohne Module.
Figur 7 zeigt eine perspektivische Ansicht einer weiteren besonders vorteilhaften Ausführungsform eines erfindungsgemäßen Behältnisses.
Figur 8 zeigt schematisch eine perspektivische Ansicht einer weiteren Ausführungsform eines erfindungsgemäßen Behältnisses.
Figur 9 zeigt eine Draufsicht auf die Unterseite einer besonders vorteilhaften Ausführungsform eines Deckels für das das erfindungsgemäße Behältnis.

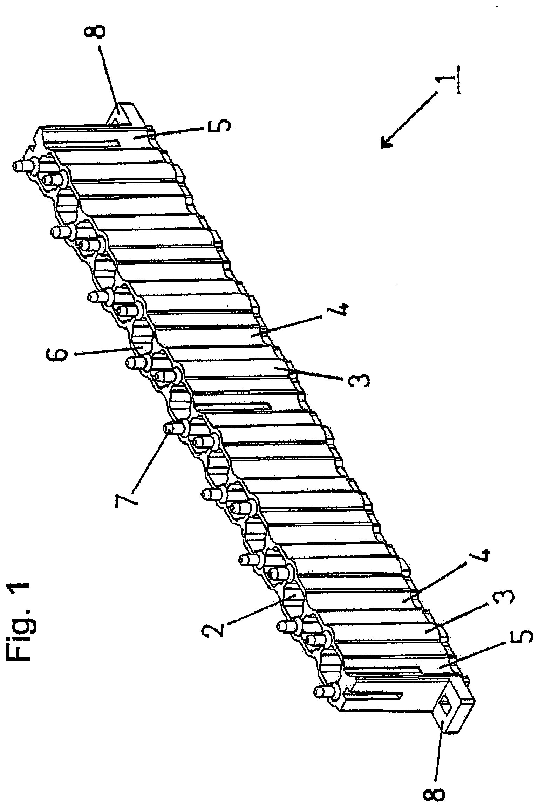

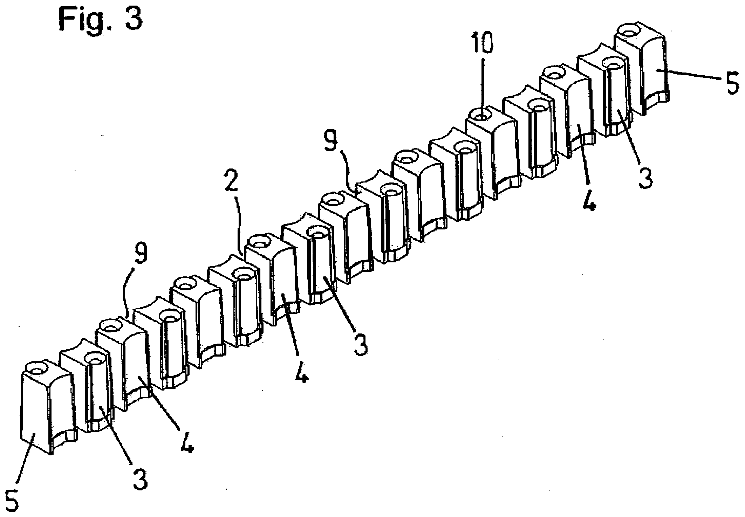

Figur 1 zeigt eine perspektivische Ansicht einer besonders vorteilhaften Ausführungsform eines erfindungsgemäßen Behältnisses. Das Behältnis 1 umfasst 16 Reaktionsräume 2, die im vorliegenden Beispiel in einer Reihe (x = 1) unmittelbar nebeneinander angeordnet sind. Jeder Reaktionsraum 2 weist zwei gegenüberliegend angeordnete Elektroden 3, 4, 5 auf, deren Form und Anordnung anhand der Figur 3 näher erläutert wird. Die jeweils zwischen zwei Reaktionsräumen 2 angeordneten Elektroden 3, 4 sind gemeinsame Elektroden 3, 4, d. h. diese Elektroden 3, 4 stehen mit den Innenräumen 6 der beiden benachbarten Reaktionsräume 2 in Verbindung. Dadurch, dass jede zwischen zwei Reaktionsräumen 2 angeordnete Elektrode 3, 4 eine gemeinsame Elektrode 3, 4 ist, umfasst das erfindungsgemäße Behältnis 1 im vorliegenden Ausführungsbeispiel insgesamt 17 Elektroden. Zusätzlich zu den gemeinsamen Elektroden 3, 4 ist an den äußeren Enden des Behältnisses 1 jeweils eine einzelne Elektrode 5 angeordnet. Das erfindungsgemäße Behältnis 1 umfasst also 16 Reaktionsräume 2 (n = 16) und 17 Elektroden 3, 4, 5 (n + x = 17). Wäre jeder Reaktionsraum mit einem separaten Elektrodenpaar versehen, so würde ein entsprechendes Behältnis mit 16 Reaktionsräumen 32 Elektroden umfassen. Durch die erfindungsgemäße Lösung umfasst das erfindungsgemäße Behältnis 1 aber nur 17 Elektroden 3, 4, 5, so dass die Anzahl der Elektroden im vorliegenden Ausführungsbeispiel um 15 Elektroden reduziert werden konnte. Im vorliegenden Ausführungsbeispiel ist jede Elektrode 3, 4, 5 mit einem Kontaktelement 7 versehen, welches die elektrische Kontaktierung der Elektroden ermöglicht. Die Kontaktelemente 7 sind im vorliegenden Ausführungsbeispiel Metallstifte, die mit dem Elektrodenmaterial umspritzt wurden. Die Kontaktelemente 7 ragen nach oben aus dem Behältnis 1 heraus, so dass die Elektroden 3, 4, 5 von oben kontaktiert werden können.

Dies hat den Vorteil, dass die Unterseite des Behältnisses 1 frei bleibt, so dass die Unterseite des Behältnisses 1 beispielsweise für optische Messungen zugänglich ist. Das erfindungsgemäße Behältnis 1 kann entweder als einzelnes Behältnis verwendet oder aber als ein Modul innerhalb einer Modulanordnung eingesetzt werden. Wird das erfindungsgemäße Behältnis 1 als Modul verwendet, so kann es mittels der Befestigungselemente 8, die jeweils an den schmalen Seiten des Behältnisses 1 angeformt sind, in einem Rahmenelement befestigt werden. Auf diese Weise können mehrere Behältnisse miteinander kombiniert werden, so dass insgesamt ein Behältnis mit einer Vielzahl von Reaktionsräumen, beispielsweise 96 oder 384 Reaktionsräume, zur Verfügung gestellt werden kann.

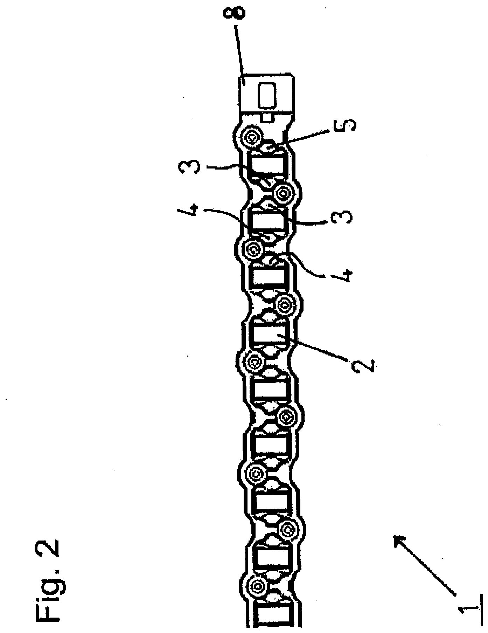

Figur 2 zeigt eine Draufsicht auf das erfindungsgemäße Behältnis 1 gemäß Figur 1. Es wird in dieser Darstellung deutlich, dass die jeweils zwischen zwei Reaktionsräumen 2 angeordneten Elektroden 3, 4 gemeinsame Elektroden 3, 4 für die beiden benachbarten Reaktionsräume 2 darstellen. Durch diese Anordnung kann das erfindungsgemäße Behältnis 1 sehr kompakt gebaut werden, wobei die Anzahl der Elektroden 3, 4, 5 bzw. die Anzahl der Kontaktelemente 7 deutlich reduziert ist. Es wird hier ferner deutlich, dass die Kontaktelemente 7 versetzt angeordnet sind, d. h. sich zickzackförmig über die Länge des Behältnisses 1 erstrecken, so dass sie den größtmöglichen Abstand zueinander einnehmen. Durch die Maximierung des Abstandes der Kontaktelemente zueinander wird erreicht, dass die Wahrscheinlichkeit von elektrischen Überschlägen zwischen zwei Kontaktelementen minimiert wird und/oder sich die maximal zulässige Pulsspannung vergrößert.

Figur 3 zeigt eine perspektivische Ansicht der Anordnung der Elektroden 3, 4, 5 des Behältnisses 1 gemäß der Figuren 1 und 2. Die Elektroden 3, 4, 5 sind in einer Reihe angeordnet, wobei die jeweils einander zugewandten Oberflächen 9 benachbarter Elektroden 3, 4, 5 einen dazwischen liegenden Reaktionsraum 2 bilden, durch den bei Anlegen einer elektrischen Spannung an die Elektroden 3, 4, 5 ein elektrischer Strom fließen kann. Die den jeweiligen Reaktionsraum 2 bildenden Oberflächen 9 der Elektroden 3, 4, 5 sind flach ausgebildet und jeweils annähernd, d. h. im Rahmen der Fertigungstoleranzen, planparallel angeordnet. Die Elektroden bestehen aus einem elektrisch leitfähigen Material, vorzugsweise aus einem Polymer, das mit einem elektrisch leitfähigen Stoff dotiert ist. Besonders bevorzugt sind Elektroden aus spritzgussfähigem Polymer, beispielsweise Polycarbonat, das mit Kohlefasern und/oder Graphit dotiert ist. Die Elektroden werden vorzugsweise im Spitzgussverfahren in ein geeignetes Spritzgusswerkzeug gespritzt. Dabei werden die Elektroden vorzugsweise nach dem Einspritzen eines weiteren Polymers, vorzugsweise transparentem Polycarbonat, in das Spritzgusswerkzeug in einem Arbeitsgang um Kontaktelemente herumgespritzt, beispielsweise Metallstifte, die in die in dieser Abbildung frei gebliebenen Bereichen 10 in das Spritzgusswerkzeug eingelegt wurden.

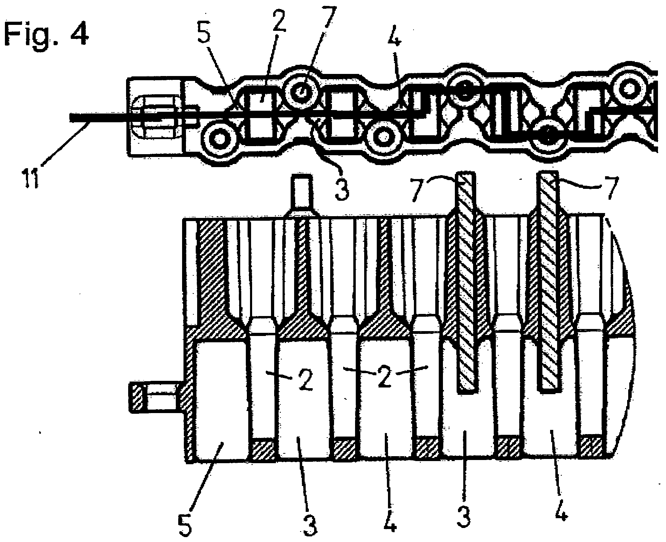

Figur 4 zeigt einen Querschnitt durch einen Teil des erfindungsgemäßen Behältnisses 1 gemäß der Figuren 1 und 2, wobei die obere Abbildung anhand der Schnittlinie 11 die Schnittführung verdeutlicht. Es wird hier nochmals deutlich, dass die gemeinsamen Elektroden 3, 4 jeweils zwischen den Reaktionsräumen 2 angeordnet sind. Die Elektroden 3, 4, 5 (hier weiß dargestellt) bestehen dabei vorzugsweise aus einem leitfähigen Polymer, beispielsweise Polycarbonat, das mit Kohlefasern und Graphit dotiert ist. Die übrigen Bereiche des Behältnisses 1 (hier schraffiert dargestellt) bestehen vorzugsweise ebenfalls aus einem Polymer, vorzugsweise aus transparentem Polycarbonat. Die Kontaktelemente 7 sind in die beiden Polymere eingespritzt und ragen nach oben aus dem Behältnis 1 heraus, so dass die Elektroden 3, 4, 5 von oben kontaktiert werden können. Die Kontaktelemente 7 sind stiftförmig ausgebildet und haben vorzugsweise einen runden Querschnitt. Die Kontaktelemente 7 sollten mit mindestens 10 % ihrer Länge, vorzugsweise 50 - 60 %, in die Polymere eingebettet sein. Die Kontaktfläche der Kontaktelemente 7 innerhalb des leitfähigen Polymers sollte vorzugsweise mindestens 5 mm², vorzugsweise 6 - 8 mm², umfassen.

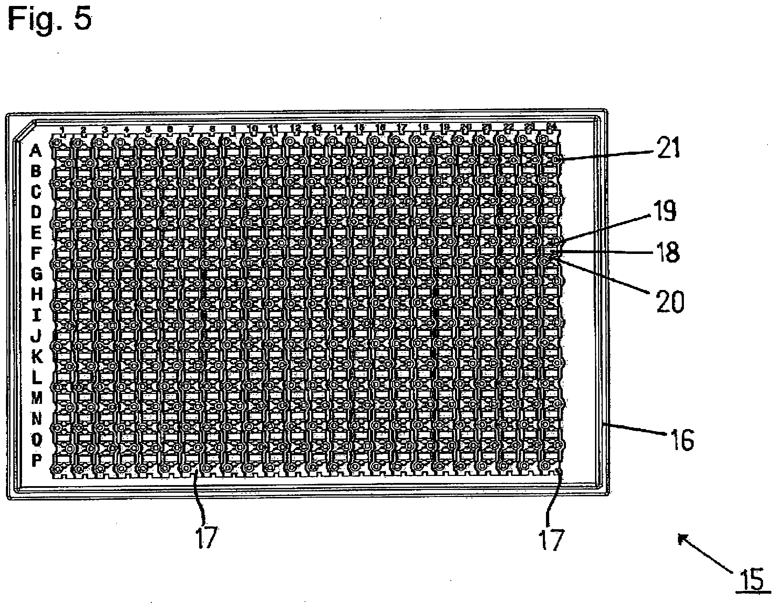

Figur 5 zeigt eine Draufsicht auf eine besondere Ausführungsform eines erfindungsgemäßen Behältnisses. Das Behältnis 15 besteht aus einem Rahmenelement 16, in dem 24 Module 17 befestigt sind. Jedes Modul 17 umfasst 16 Reaktionsräume 18, die jeweils in einer Reihe angeordnet sind. Insgesamt umfasst das Behältnis 15 also 384 Reaktionsräume 18. Die Module 17 entsprechen dabei jeweils dem Behältnis 1 gemäß Figur 1, d. h. jeder Reaktionsraum 18 weist zwei Elektroden 19, 20 auf, die jeweils mit einem nach oben aus dem Behältnis 15 herausragenden Kontaktelement 21 versehen sind. Das erfindungsgemäße Behältnis 15 eignet sich insbesondere für Elektrotransfektionen im Hochdurchsatzverfahren (high throughput screening).

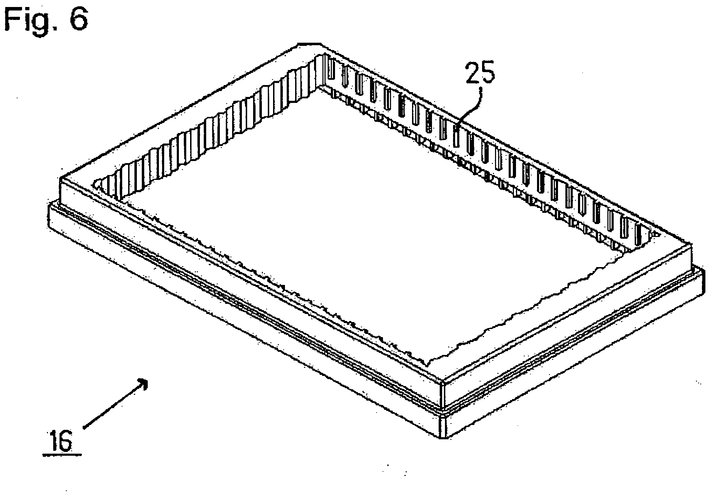

Figur 6 zeigt eine perspektivische Ansicht des Rahmenelements 16 gemäß Figur 5 ohne Module. Das Rahmenelement 16 ist ein Kunststoffspritzgussteil, das vorzugsweise aus Polystyrol besteht. Die Innenseiten der Längsseiten des Rahmenelements 16 weisen Führungsmittel 25 auf, die der Führung bzw. Zentrierung der Module 17 gemäß Figur 5 dienen. Die Module können beispielsweise durch Heißverstemmen im dem Rahmenelement 16 befestigt werden. Dazu müssen Rahmenelemente im Spritzguss gefertigt werden, die pro zu befestigendem Modul auf jeder Seite einen Kunststoffpin aufweisen. Die Module wiederum weisen an beiden Schmalseiten Ösen auf, die an die Größe der Kunststoffpins angepasst sind. So kann man die Module in die Rahmen einlegen. Die Pins werden dann mittels eines passenden Werkzeugs erhitzt und es kommt zum Formschluss zwischen Pin und Öse.

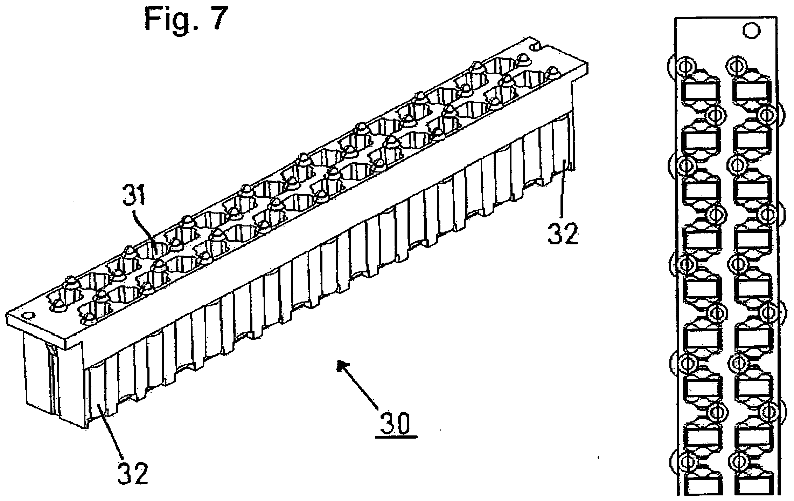

Figur 7 zeigt eine perspektivische Ansicht einer weiteren besonders vorteilhaften Ausführungsform eines erfindungsgemäßen Behältnisses. Das Behältnis 30 entspricht in seinem Aufbau im Wesentlichen dem Behältnis 1 gemäß Figur 1. Im Unterschied zum Behältnis 1 gemäß Figur 1 umfasst das Behältnis 30 zwei Reihen (x = 2) von Reaktionsräumen 31. Jede Reihe umfasst dabei sechzehn Reaktionsräume 31, so dass das Behältnis 30 insgesamt 32 Reaktionsräume (n = 32) umfasst. Jede Reihe des Behältnisses 30 umfasst siebzehn Elektroden, so dass das Behältnis 30 insgesamt 34 Elektroden 32 (n + x = 34) umfasst. Wäre jeder Reaktionsraum mit einem separaten Elektrodenpaar versehen, so würde ein entsprechendes Behältnis mit 32 Reaktionsräumen 64 Elektroden umfassen. Durch die erfindungsgemäße Lösung umfasst das erfindungsgemäße Behältnis 30 aber lediglich 34 Elektroden 32, so dass die Anzahl der Elektroden im vorliegenden Ausführungsbeispiel um 15 Elektroden reduziert ist. Auf diese Weise kann das aktive Reaktionsraumvolumen bei gegebener Größe maximiert werden.

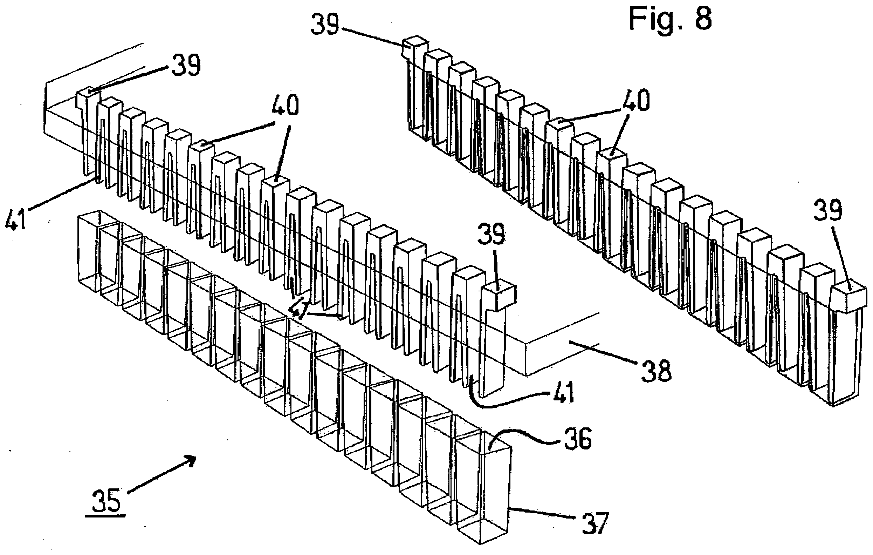

Figur 8 zeigt schematisch eine perspektivische Ansicht einer weiteren Ausführungsform eines erfindungsgemäßen Behältnisses. Das erfindungsgemäße Behältnis 35 umfasst mehrere Reihen von Reaktionsräumen, von denen hier nur eine Reihe dargestellt ist. Die Reaktionsräume 36 sind jeweils durch einen Wandbereich 37 gebildet, welcher keine Elektroden aufweist. Das Behältnis 35 umfasst ferner einen Deckel 38, an dessen den Reaktionsräumen 36 zugewandten Innenseite Elektroden 39, 40 befestigt sind. Beim Aufsetzen des Deckels 38 auf die Wandbereiche 37 der Reaktionsräume 36 tauchen die Elektroden 39, 40 in die jeweiligen Reaktionsräume 36 ein. Beim Anlegen einer elektrischen Spannung an die Elektroden 39, 40 kann dann elektrischer Strom durch die Reaktionsräume 36 fließen. Die jeweils an den beiden Enden einer Reihe angeordneten Elektroden 39 sind Einzelelektroden, die jeweils nur in einen Reaktionsraum 36 eintauchen. Die dazwischen liegenden Elektroden 40 sind dagegen gemeinsame Elektroden, die jeweils zwei beinartige Fortsätze 41 aufweisen. Jeweils einer der beinartigen Fortsätze 41 jeweils benachbarter Elektroden 40 taucht in einen gemeinsamen Reaktionsraum 36 ein, während die beiden beinartigen Fortsätze 41 jeder Elektrode 40 in jeweils benachbarte Reaktionsräume 36 eintauchen (siehe Darstellung rechts oben, ohne Deckel). Auf diese Weise bilden die Elektroden 40 jeweils eine gemeinsame Elektrode für zwei benachbarte Reaktionsräume 36. Jeder Reaktionsraum 36 umfasst folglich ein Elektrodenpaar, dass entweder aus zwei beinartigen Fortsätzen 41 von zwei Elektroden 40 oder einem beinartigen Fortsatz 41 von einer Elektrode 40 und einem beinartigen Fortsatz 41 von einer einzelnen Elektrode 39 besteht. Abgebildet ist in dieser Darstellung eine Reihe (x = 1) des erfindungsgemäßen Behältnisses 35 mit 16 Reaktionsräumen 36 (n = 16). Das Behältnis 35 umfasst ferner 17 Elektroden 39, 40 (n + x = 16 + 1 = 17) und weist somit eine gegenüber bereits bekannten Behältnissen deutlich reduzierte Elektrodenzahl auf.

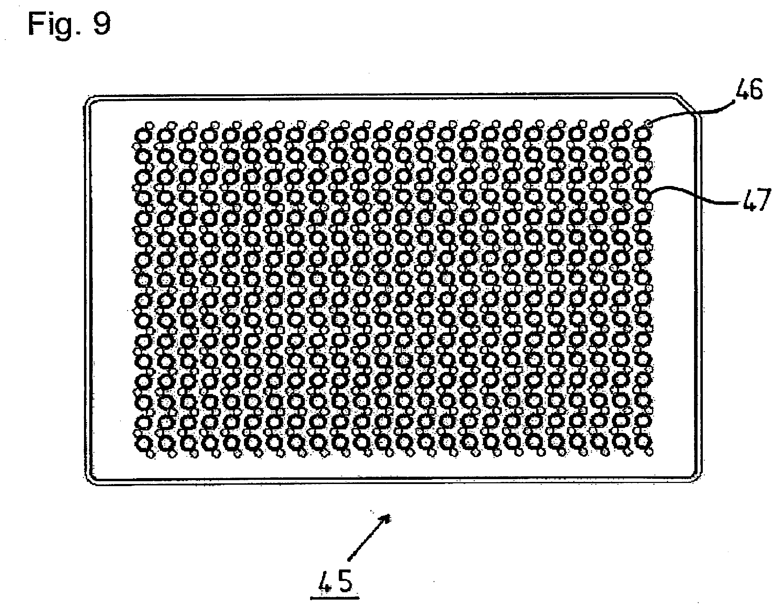

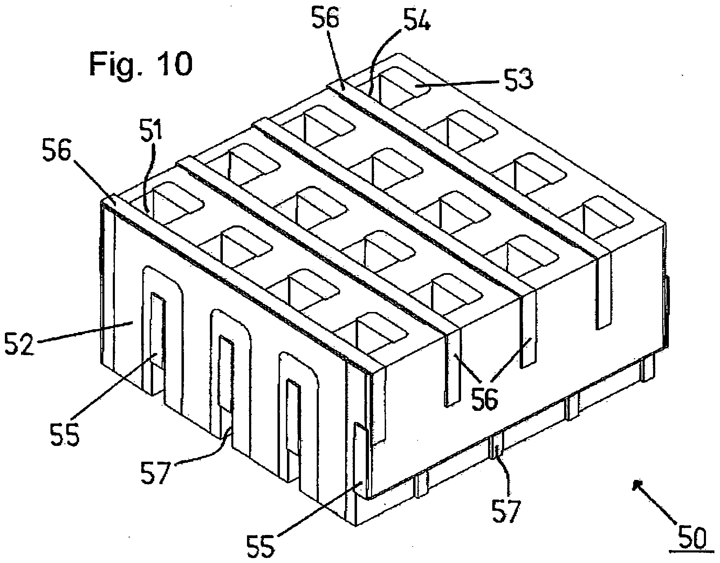

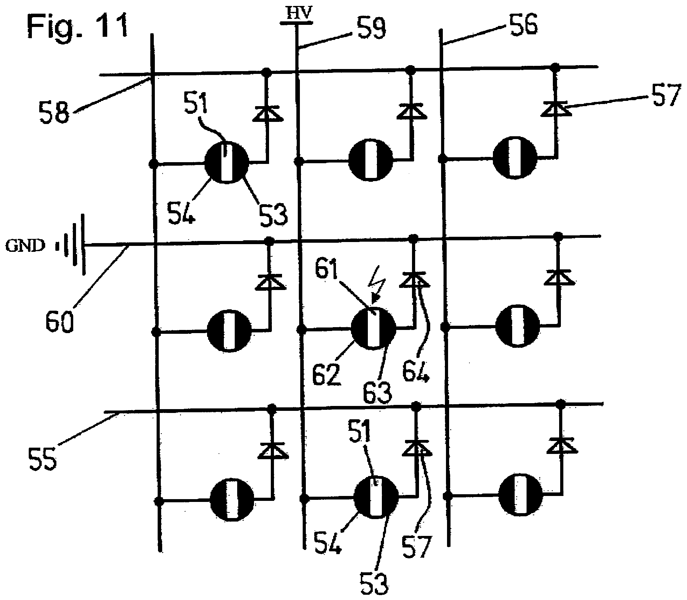

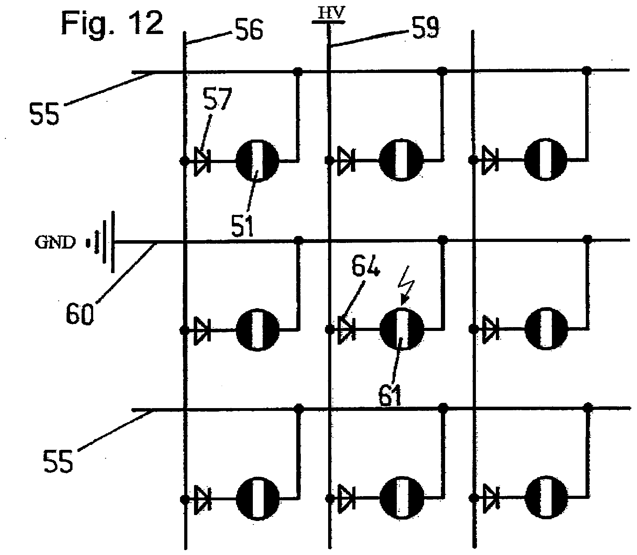

Figur 9 zeigt eine Draufsicht auf die Unterseite einer besonders vorteilhaften Ausführungsform eines Deckels für das das erfindungsgemäße Behältnis. Der Deckel 45 kann beispielsweise der Abdeckung der Reaktionsräume des erfindungsgemäßen Behältnisses 15 gemäß Figur 5 dienen. Der Deckel 45 weist eine der Anzahl der Kontaktelemente entsprechende Anzahl von Löchern 46 auf, durch welche die Kontaktelemente kontaktiert werden können. Der Deckel weist an seiner Unterseite, die dem Behältnis zugewandt ist, kreisförmige Dichtungsringe 47 auf, welche die Reaktionsräume des Behältnisses bei aufgesetztem Deckel 45 abdichten. Auf diese Weise können Kreuzkontaminationen durch Spritzen der in den Reaktionsräumen befindlichen Zellsuspension vermieden werden. Anstelle der Dichtungsringe 47 können auch einfache Erhebungen oder Wulste vorgesehen sein, sofern diese die Reaktionsräume gegeneinander abdichten können.

### Bezugszeichenliste:

- 1: Behältnis
- 2: Reaktionsraum
- 3: Elektrode
- 4: Elektrode
- 5: Elektrode
- 6: Innenraum
- 7: Kontaktelement
- 8: Befestigungselement
- 9: Oberfläche
- 10: Bereiche
- 11: Schnittlinie
- 15: Behältnis
- 16: Rahmenelement
- 17: Modul
- 18: Reaktionsraum
- 19: Elektrode
- 20: Elektrode
- 21: Kontaktelement
- 25: Führungsmittel
- 30: Behältnis
- 31: Reaktionsraum
- 32: Elektrode
- 35: Behältnis
- 36: Reaktionsraum
- 37: Wandbereich
- 38: Deckel
- 39: Elektrode
- 40: Elektrode
- 41: Fortsatz
- 45: Deckel
- 46: Löcher
- 47: Dichtungsring

## Patentansprüche

1. Behältnis (1, 15, 30, 35) mit mindestens drei Reaktionsräumen (2, 18, 31, 36), die jeweils mindestens ein Elektrodenpaar (3, 4, 5, 19, 20, 32, 39, 40) zum Anlegen einer elektrischen Spannung zur Erzeugung eines elektrischen Feldes innerhalb des Reaktionsraums (2, 18, 31, 36) aufweisen und die geometrisch in mindestens einer Reihe angeordnet und/oder elektrisch in mindestens einer Reihe geschaltet sind, wobei mindestens eine Elektrode eines Reaktionsraums (2, 18, 31, 36) eine gemeinsame Elektrode (3, 4, 40) mit mindestens einem anderen Reaktionsraum (2, 18, 31, 36) ist, **dadurch gekennzeichnet, dass** n + x Elektroden (3, 4, 5, 19, 20, 32, 39, 40) vorgesehen sind, wobei n die Anzahl der Reaktionsräume (2, 18, 31, 36) mit n ≥ 3 und x die Anzahl der Reihen mit x ≥ 1 ist.

2. Behältnis nach Anspruch 1, **dadurch gekennzeichnet, dass** innerhalb der Reihe geometrisch mindestens eine gemeinsame Elektrode (3, 4, 40) zumindest teilweise zwischen zwei benachbarten Reaktionsräumen (2, 18, 31, 36) angeordnet ist.

3. Behältnis nach Anspruch 1 oder 2, **dadurch gekennzeichnet, dass** die Elektroden (3, 4, 5, 19, 20, 32, 39, 40) mit Kontaktelementen (7, 21) versehen sind, die zum Anlegen der elektrischen Spannung elektrisch kontaktierbar sind.

4. Behältnis nach Anspruch 3, **dadurch gekennzeichnet, dass** die gemeinsame Elektrode (3, 4, 40) der beiden Reaktionsräume (2, 18, 31, 36) mit einem Kontaktelement (7, 21) versehen ist.

5. Behältnis nach Anspruch 3 oder 4, **dadurch gekennzeichnet, dass** die Kontaktfläche der Kontaktelemente (7, 21) zu den Elektroden (3, 4, 5, 19, 20, 32, 39, 40), die sich an oder innerhalb der Elektroden (3, 4, 5, 19, 20, 32, 39, 40) befindet, eine Fläche von mindestens 5 mm², vorzugsweise mindestens 6 mm², besonders bevorzugt mindestens 7 mm², insbesondere mindestens 8 mm², aufweist.

6. Behältnis nach einem der Ansprüche 3 bis 5, **dadurch gekennzeichnet, dass** die Kontaktelemente (7, 21) stift-, nadel- oder schraubenförmig ausgebildet sind und/oder einen zumindest annähernd runden Querschnitt aufweisen.

7. Behältnis nach einem der Ansprüche 3 bis 6, **dadurch gekennzeichnet, dass** die Kontaktelemente (7, 21) an der Oberseite der Elektroden (3, 4, 5, 19, 20, 32, 39, 40) angeordnet sind und/oder nach oben aus den Elektroden (3, 4, 5, 19, 20, 32, 39, 40) herausragen.

8. Behältnis nach einem der Ansprüche 1 bis 7, **dadurch gekennzeichnet, dass** die Elektroden (3, 4, 5, 19, 20, 32, 39, 40) nach unten über den Boden des Behältnisses (1, 15, 30, 35) und/oder den Wandbereich der Reaktionsräume (2, 18, 31, 36) hinausragen.

9. Behältnis nach einem der Ansprüche 1 bis 8 beinhaltend einen Deckel (45), **dadurch gekennzeichnet, dass** die Elektroden (3, 4, 5, 19, 20, 32) über Kontaktstellen elektrisch kontaktierbar sind und der Deckel (45) die Reaktionsräume (2,18, 31) abdeckt, wobei der Deckel (45) eine der Anzahl der Kontaktstellen entsprechende Anzahl von Löchern (46) aufweist.

10. Behältnis nach Anspruch 9, **dadurch gekennzeichnet, dass** der Deckel (45) n + x Löcher (46) aufweist, wobei n die Anzahl der Reaktionsräume (2, 18, 31) mit n ≥ 3 und x die Anzahl der Reihen mit x ≥ 1 ist.

11. Behältnis nach einem der Ansprüche 1 bis 8, **dadurch gekennzeichnet, dass** das Behältnis unlösbar mit mindestens einem Transponder verbunden ist.

12. Behältnis nach Anspruch 11, **dadurch gekennzeichnet, dass** mindestens ein Reaktionsraum von einem Wandbereich begrenzt ist, in den der Transponder integriert ist.

13. Behältnis nach Anspruch 11 oder 12, **dadurch gekennzeichnet, dass** der Transponder ein RFID-Transponder ist.

14. Verfahren zur Herstellung des Behältnisses nach einem der Ansprüche 11 bis 13, **dadurch gekennzeichnet, dass** das Behältnis im Spritzgussverfahren hergestellt wird, wobei der Transponder von zumindest einem Polymer umspritzt wird.

## Claims

1. Container (1, 15, 30, 35) with at least three reaction spaces (2, 18, 31, 36) which each have at least one pair of electrodes (3, 4, 5, 19, 20, 32, 39, 40) for applying an electric voltage for generating an electric field within the reaction space (2, 18, 31, 36) and which are arranged geometrically in at least one row and/or electrically connected in at least one row, wherein at least one electrode of a reaction space (2, 18, 31, 36) is a common electrode (3, 4, 40) with at least one other reaction space (2, 18, 31, 36), **characterised in that** n + x electrodes (3, 4, 5, 19, 20, 32, 39, 40) are provided, wherein n is the number of reaction spaces (2, 18, 31, 36), with n ≥ 3, and x is the number of rows, with x ≥ 1.

2. Container according to claim 1, **characterised in that** at least one common electrode (3, 4, 40) is geometrically arranged at least partially between two adjacent reaction spaces (2, 18, 31, 36) within the row.

3. Container according to claim 1 or 2, **characterised in that** the electrodes (3, 4, 5, 19, 20, 32, 39, 40) are provided with contact elements (7, 21) which can be electrically contacted for applying the electric voltage.

4. Container according to claim 3, **characterised in that** the common electrode (3, 4, 40) of the two reaction spaces (2, 18, 31, 36) is provided with a contact element (7, 21).

5. Container according to claim 3 or 4, **characterised in that** the contact surface of the contact elements (7, 21) to the electrodes (3, 4, 5, 19, 20, 32, 39, 40), which is located on or within the electrodes (3, 4, 5, 19, 20, 32, 39, 40), has an area of at least 5 mm², preferably at least 6 mm², particularly preferably at least 7 mm², and in particular at least 8 mm².

6. Container according to any one of claims 3 to 5, **characterised in that** the contact elements (7, 21) are constructed in a pin shape, needle shape or screw shape and/or have an at least approximately round cross section.

7. Container according to any one of claims 3 to 6, **characterised in that** the contact elements (7, 21) are arranged on the upper side of the electrodes (3, 4, 5, 19, 20, 32, 39, 40) and/or project upwardly out of the electrodes (3, 4, 5, 19, 20, 32, 39, 40).

8. Container according to any one of claims 1 to 7, **characterised in that** the electrodes (3, 4, 5, 19, 20, 32, 39, 40) project downwardly beyond the bottom of the container (1, 15, 30, 35) and/or the wall region of the reaction spaces (2, 18, 31, 36).

9. Container according to any one of claims 1 to 8 including a lid (45), **characterised in that** the electrodes (3, 4, 5, 19, 20, 32) can be electrically contacted by means of contact points and the lid (45) covers the reaction spaces (2, 18, 31), wherein the lid (45) has a number of holes (46) corresponding to the number of contact points.

10. Container according to claim 9, **characterised in that** the lid (45) has n + x holes (46), wherein n is the number of the reaction spaces (2, 18, 31), with n ≥ 3, and x is the number of rows, with x ≥ 1.

11. Container according to any one of claims 1 to 8, **characterised in that** the container is unreleasably connected to at least one transponder.

12. Container according to claim 11, **characterised in that** at least one reaction space is delimited by a wall region, into which the transponder is integrated.

13. Container according to claim 11 or 12, **characterised in that** the transponder is a RFID transponder.

14. Method for producing the container according to any one of claims 11 to 13, **characterised in that** the container is produced by injection molding, wherein the transponder is overmolded by at least one polymer.

## Revendications

1. Contenant (1, 15, 30, 35) avec au moins trois chambres de réaction (2, 18, 31, 36) qui comportent chacune au moins une paire d'électrodes (3, 4, 5, 19, 20, 32, 39, 40) destinées à appliquer une tension électrique pour créer un champ électrique à l'intérieur de la chambre de réaction (2, 18, 31, 36) et qui sont géométriquement placées en au moins une série et/ou qui sont électriquement montées en au moins une série, au moins une électrode d'une chambre de réaction (2, 18, 31, 36) étant une électrode (3, 4, 40) commune avec au moins une autre chambre de réaction (2, 18, 31, 36), **caractérisé en ce que** n + x électrodes (3, 4, 5, 19, 20, 32, 39, 40) sont prévues, n étant le nombre des chambres de réaction (2, 18, 31, 36), avec n ≥ 3 et x étant le nombre de séries, avec x ≥ 1.

2. Contenant selon la revendication 1, **caractérisé en ce qu'**à l'intérieur de la série est géométriquement placée au moins une électrode (3, 4, 40) commune, au moins en partie entre deux chambres de réaction (2, 18, 31, 36) voisines.

3. Contenant selon la revendication 1 ou 2, **caractérisé en ce que** les électrodes (3, 4, 5, 19, 20, 32, 39, 40) sont munies d'éléments de contact (7, 21) qui peuvent être contactés électriquement, pour l'application de la tension électrique.

4. Contenant selon la revendication 3, **caractérisé en ce que** l'électrode (3, 4, 40) commune des deux chambres de réaction (2, 18, 31, 36) est munie d'un élément de contact (7, 21).

5. Contenant selon la revendication 3 ou 4, **caractérisé en ce que** la surface de contact des éléments de contact (7, 21) vers les électrodes (3, 4, 5, 19, 20, 32, 39, 40) qui se trouve sur ou à l'intérieur des électrodes (3, 4, 5, 19, 20, 32, 39, 40) présente une surface d'au moins 5 mm², de préférence d'au moins 6 mm², de manière particulièrement préférée d'au moins 7 mm², notamment d'au moins 8 mm².

6. Contenant selon l'une quelconque des revendications 3 à 5, **caractérisé en ce que** les éléments de contact (7, 21) sont conçus en forme de tige, d'aiguille ou d'hélice et/ou présentent une section transversale au moins approximativement ronde.

7. Contenant selon l'une quelconque des revendications 3 à 6, **caractérisé en ce que** les éléments de contact (7, 21) sont placés sur la face supérieure des électrodes (3, 4, 5, 19, 20, 32, 39, 40) et/ou ressortent vers le dessus hors des électrodes (3, 4, 5, 19, 20, 32, 39, 40).

8. Contenant selon l'une quelconque des revendications 1 à 7, **caractérisé en ce que** les électrodes, 4, 5, 19, 20, 32, 39, 40) ressortent vers le bas, au-delà du fond inférieur du contenant (1, 15, 30, 35) et ou de la zone de paroi des chambres de réaction (2, 18, 31, 36).

9. Contenant selon l'une quelconque des revendications 1 à 8, comprenant un couvercle (45), **caractérisé en ce que** les électrodes (3, 4, 5, 19, 20, 32) peuvent être mises électriquement en contact par l'intermédiaire de zones de contact et **en ce que** le couvercle (45) recouvre les chambres de réaction (2, 18, 31), le couvercle (45) comportant un nombre de trous (46) correspondant au nombre des zones de contact.

10. Contenant selon la revendication 9, **caractérisé en ce que** le couvercle (45) comporte n + x trous (46), n étant le nombre des chambres de réaction (2, 18, 31), avec n ≥ 3 et x étant le nombre de séries, avec x ≥ 1.

11. Contenant selon l'une quelconque des revendications 1 à 8, **caractérisé en ce que** le contenant est assemblé de manière inamovible avec au moins un transpondeur.

12. Contenant selon la revendication 11, **caractérisé en ce que** l'au moins une chambre de réaction est délimitée par une zone de paroi, dans laquelle le transpondeur est intégré.

13. Contenant selon la revendication 11 ou 12, **caractérisé en ce que** le transpondeur est un transpondeur RFID.

14. Procédé destiné à fabriquer un contenant selon l'une quelconque des revendications 11 à 13, **caractérisé en ce qu'**on fabrique le contenant par procédé de moulage par injection, sachant qu'on enrobe le transpondeur par injection d'au moins un polymère.
